# EUROPÄISCHE PATENTSCHRIFT

(11) **EP 2 862 654 B1**
(45) Veröffentlichungstag und Bekanntmachung des Hinweises auf die Patenterteilung: **01.09.2021**
(21) Anmeldenummer: 14188431.2
(22) Anmeldetag: 10.10.2014
(51) Int. Cl.: A61B 17/16

(54) **Bohrsystem**
Drilling system
Système perforateur

(30) Priorität: 10.10.2013 DE 102013111200
(43) Veröffentlichungstag der Anmeldung: 22.04.2015
(60) Teilanmeldung: 20212231.3
(73) Patentinhaber: Fraunhofer-Gesellschaft zur Förderung der angewandten Forschung e.V., 80686 München (DE)
(72) Erfinder: Nakajima, Kiyoharu, 75175 Pforzheim (DE); Schwarz, Oliver, 70499 Stuttgart (DE); Miklosovic, Miroslav, 70597 Stuttgart (DE)
(74) Vertreter: Mammel und Maser

(56) Entgegenhaltungen:
- EP-A2- 1 110 513
- DE-U1-202005 011 940
- DE-U1-202009 005 880
- JP-A- H06 155 401
- US-A- 2 970 660
- US-A- 3 383 747
- US-A- 4 867 251
- A. Ellery, et al.: "BIONICS & SPACE SYSTEM DESIGN (AO/1-4469/03/NL/SFe) CASE STUDY 2 Asteroid Micro-Penetrator withBiomimetic Drill", ESA WEBSITE , 1. Juni 2005 (2005-06-01), Seiten i-131, XP002736130, Gefunden im Internet: URL:http://www.esa.int/gsp/ACT/doc/BIO/ACT -RPT-BIO-GSP-04L27a-BiomimeticsSpaceSystem Design%20-%20CaseStudy2%20-%20BiomimeticDr ill.pdf [gefunden am 2015-02-16]
- None

## Beschreibung

Die Erfindung betrifft ein Bohrsystem mit einem Bohrinstrument, welches einen Antrieb aufweist, der ein Bohrwerkzeug antreibt sowie ein Verfahren zum Antreiben des Bohrwerkzeuges.

Allgemein sind Bohrsysteme bekannt, bei welchen die Bohrer eine rotierende Schneidbewegung ausführen. Dies erfolgt zumeist über Spiralbohrer. Solche Spiralbohrer können in der Medizintechnik nur beschränkt eingesetzt werden. Beispielsweise kann nicht direkt durch die Haut gebohrt werden, da diese sich um den Bohrer wickelt. Zudem tritt bei solchen kreisförmigen Schneidbewegungen mittels Spiralbohrer oft der Nachteil auf, dass eine örtliche Erhitzung entsteht, die unerwünscht ist.

Aus der DE 20 2005 011 940 U1 ist ein geschlitzter Bohrer bekannt, der eine rotierende Schneidwirkung ausübt. Durch die Ausbildung eines Schlitzes von Bohrkopf in Richtung auf den Bohrschaft wird die Herstellung eines konusförmigen Bohrlochs ermöglicht.

Aus der DE 20 2009 005 880 U1 ist ein Werkzeug zur Herstellung von Nuten in einem Werkstück bekannt, welches aus einer auf- und abbewegbaren Räumnadel besteht, die entlang einer Führungsstange mit einem Vorschubkeil in einer Auf- und Abbewegung geführt wird. Die Räumnadel und die Führungsstangen sind im Zuge eines Vorschubes relativ zueinander in Längsrichtung verschiebbar, um die Nut einzubringen.

Aus der Veröffentlichung Bionics & Space System Design (AO 1/4469/03/NL/SFe Case Study 2 - Asterioid Micro-Penetrater with Biometic Drill der University of Surrey, Großbritannien) ist ein alternatives Bohrsystem bekannt, welches die Merkmale der Oberbegriffe der unabhängigen Ansprüche 1 und 16 aufweist und einen Bohrer mit zwei Bohrspitzen umfasst, die gegenläufig zueinander linear bewegt angetrieben sind. Die Bohrspitze weist eine kreisrunde Geometrie auf. Ein solches Bohrinstrument ist für den Einsatz im Weltall zur Entnahme von Gesteinsproben vorgesehen.

Dieses Bohrinstrument mit den beiden Bohrspitzen beziehungsweise einem geteilten Bohrer weist den Nachteil auf, dass eine unkontrollierte und ungewollte Bewegung auf das Werkstück ausgeübt und eine hohe Axialkraft zum Bohren benötigt wird.

Der Erfindung liegt die Aufgabe zugrunde, ein Bohrsystem zu schaffen, welches nach dem Pendelhubbohrprinzip arbeitet und eine kontrollierte Bohrbewegung mit einer geringen Axialkraft beim Bohren ermöglicht.

Diese Aufgabe wird erfindungsgemäß durch ein Bohrsystem gemäß dem Anspruch 1 bzw. durch ein Bohrwerkzeug gemäß dem Anspruch 16 gelöst. Bei dem Bohrsystem des Anspruchs 1 wird ein Bohrwerkzeug mit drei oder mehr Bohrsegmenten eingesetzt, welche einen Bohrkopf bilden, wobei die einzelnen Bohrsegmente in einer vorbestimmten aufeinanderfolgenden Reihenfolge nach dem Pendelhubprinzip angetrieben werden. Dadurch kann eine geringere Axialkraft genügen, um eine Vorwärtsbewegung der Bohrspitze in das zu bohrende Material beziehungsweise in dem zu bohrenden Werkstoff zu erzeugen. Erstaunlicherweise hat sich gezeigt, dass bereits ein Bohrwerkzeug mit drei Bohrsegmenten, die jeweils eine Bohrspritze umfassen, um eine gemeinsame Bohrspitze des Bohrwerkzeugs zu bilden, genügt, um eine erhebliche Verringerung der Druckkraft beziehungsweise Axialkraft für eine Pendelhubbewegung zum Antrieb der jeweiligen Bohrsegmente zur Erzeugung einer Bohrung zu erzielen. Darüber hinaus lassen sich Bohrungen mit verschiedenen und von runden Bohrungen abweichende Querschnittskonturen herstellen.

Darüber hinaus weist ein solches Bohrsystem den Vorteil auf, dass Bohrungen in einen Werkstoff oder in ein Material eingebracht werden könne, welche durch Spiralbohrungen nicht möglich sind. Beispielsweise kann ein solches Bohrinstrument zum Bohren in der Haut oder dem Knochen verwendet werden. Darüber hinaus weist dieses Bohrsystem den Vorteil auf, dass während des Bohrens keine Wärme entsteht, die zu Beschädigungen des Materials oder des Werkstoffs führen könnte.

Bevorzugt ist vorgesehen, dass eine Antriebsachse des Antriebs in der Längsachse des Bohrwerkzeugs liegt und vorzugsweise ein Getriebe, insbesondere eine Taumelscheibe oder einen Kurvenantrieb, ansteuerbar ist. Dadurch kann ein mechanisch einfacher, jedoch sicherer Antrieb vorgesehen sein, um zumindest die drei Bohrsegmente in einer aufeinander folgenden Reihenfolge anzutreiben.

Eine alternative Ausgestaltung des Bohrsystems sieht vor, dass die Antriebsachse des Antriebs quer zur Längsachse des Bohrwerkzeugs liegt und das Bohrwerkzeug mit einem Getriebe, insbesondere einem Kurbelantrieb, wie beispielsweise einer Kurbelwelle, ansteuerbar ist. Dadurch kann ebenfalls eine einfache mechanische Konstruktion geschaffen werden, durch welche vorteilhafterweise die Reihenfolge der Vorwärtsbewegungen der einzelnen Bohrsegmente festgelegt ist.

Eine weitere alternative Ausgestaltung des Bohrsystems sieht vor, dass jedes Bohrsegment einzeln mit einem elektrischen, pneumatischen oder hydraulischen Antrieb ansteuerbar ist. Dabei kann sowohl eine festgelegte Reihenfolge in dem Antrieb der Bohrsegmente aufeinander vorgesehen sein als auch eine von einem im oder gegen den Uhrzeigersinn aufeinander folgenden Antrieb der einzelnen Bohrsegmente abweichende Reihenfolge sowie eine wahlweise oder zufällige Reihenfolge der Bohrsegmente möglich sein. Dadurch kann beispielsweise ein Antrieb geschaffen werden, bei welchem zwei der drei Bohrsegmente durch eine Pendelhubbewegung hin und her bewegt werden, wobei das dritte Bohrsegment lediglich einen Vorschub erfährt.

Des Weiteren ist bevorzugt vorgesehen, dass das Bohrwerkzeug des Bohrsystems als ein Bohrsegment - quer zur Längsachse betrachtet - mit einem Schneidenabschnitt und zumindest zwei Führungsabschnitte ausgebildet ist, wobei zwei an den Schneidenabschnitten angrenzende Führungsabschnitte mit korrespondierenden Führungsabschnitten der benachbarten Bohrsegmente vorzugsweise in einer Trennebene aneinander anliegen. Dadurch können sich die drei oder mehr Bohrsegmente bei Eindringen in den Werkstoff gegenseitig abstützen, so dass eine kontrollierte Bewegung der jeweiligen Bohrsegmente möglich ist.

Bevorzugt sind an den aneinander liegenden Führungsabschnitten der Bohrsegmente Führungselemente vorgesehen, durch welche die Führungsabschnitte miteinander in Verbindung stehen. Diese Führungselemente können dazu ausgebildet sein, dass die gegenläufige Axialbewegung der einzelnen Bohrsegmente geführt ist, so dass ein seitliches Ausweichen verhindert wird. Hierbei können ineinander greifende Führungselemente ausgebildet sein, die beispielsweise eine Nut- und Federverbindung, eine L-förmige Kontur oder dergleichen aufweisen.

Die Trennebene der aneinander liegenden Führungsabschnitte ist vorzugsweise im gleichen Winkel zueinander angeordnet. Bei beispielsweise drei Bohrsegmenten sind die Trennebenen bevorzugt in einem Winkel von 120° zueinander angeordnet. Dadurch können auch gleiche Kraftverhältnisse geschaffen werden.

Nach einer weiteren bevorzugten Ausführungsform weist der Schneidenabschnitt von dem einen Bohrsegment mit dem Schneidenabschnitt des weiteren Bohrsegments eine runde, ovale, dreieck- oder mehreckförmige Schneidengeometrie auf. Durch diese Ausgestaltung des drei- oder mehrteiligen Bohrwerkzeuges und deren Antrieb über die Pendelhubbewegung sind auch von einer runden Geometrie abweichende Bohrquerschnitte herstellbar. Dadurch kann sich ein weites Einsatzfeld gegenüber runden Bohrungen eröffnen, die durch rotierende Spiralbohrer hergestellt werden.

Bei einer Ausführungsform des Bohrwerkzeuges ist bevorzugt vorgesehen, dass jedes Bohrsegment ein Schneidenabschnitt und ein Führungsabschnitt aufweist, wobei die Führungsabschnitte miteinander korrespondieren und aneinander anliegen. Dadurch können sich diese Bohrsegmente gegenseitig abstützen.

Des Weiteren ist bevorzugt vorgesehen, dass der Schneidenabschnitt von dem einen Bohrsegment mit dem weiteren Bohrsegment eine ovale, dreieckförmige, mehreckförmige Schneidengeometrie aufweist. Dadurch können ganz spezifische Bohrgeometrien hergestellt werden.

Eine alternative Ausführungsform des Bohrwerkzeuges des Bohrsystems sieht vor, dass eines der Bohrsegmente einen größeren Segmentquerschnitt als das oder die weiteren Bohrsegmente aufweist. Dadurch können verschiedene Geometrien eines Bohrkopfes geschaffen werden. Insbesondere kann bei einem Bohrwerkzeug mit drei Bohrsegmenten vorgesehen sein, dass ein Segmentabschnitt des Bohrsegments 50 % des Gesamtquerschnitts und die beiden weiteren Bohrsegmente jeweils einen Segmentquerschnitt von 25 % des Gesamtquerschnitts des Bohrkopfes aufweisen. Dadurch kann beispielsweise das Bohrsegment mit dem größeren Querschnitt als Stützabschnitt dienen und die beiden Bohrsegmente mit den kleineren Segmentabschnitten die Bohrarbeiten verrichten sowie das Material abtragen.

Eine weitere bevorzugte Ausführungsform des Bohrsystems sieht vor, dass das Bohrwerkzeug für jedes Bohrsegment eine Lanze umfasst, an der zumindest der Schneidenabschnitt lösbar angeordnet ist. Dadurch können beispielsweise zwei Prozesse miteinander verbunden werden: Zum einen Bohren und Schrauben und zum anderen Bohren und Verkeilen oder Bohren und Dübeln.

Bevorzugt ist der drei- oder mehrteilige Bohrkopf des Bohrwerkzeugs mit einem Außengewinde versehen, welches sich über den Bohrkopf erstreckt. Dadurch kann zunächst ein Bohren ermöglicht sein und anschließend mit einer Drehbewegung eine Schraubbewegung der Bohrspitze erzeugt werden, wobei insbesondere durch die lösbare Lanze von der Bohrspitze die Bohrsegmente in dem Material verbleiben können. Dadurch kann eine zusätzliche Verankerung erzielt werden.

Eine alternative Ausführungsform der Erfindung sieht vor, dass der drei- oder mehrteilige Bohrkopf zur zentrischen Aufnahme eines Spreizelementes ausgebildet ist. Beispielsweise kann nach dem Eindringen des Bohrwerkzeuges in das Material die Lanze wiederum von der Bohrspitze beziehungsweise dem Schneidenabschnitt gelöst und darauf folgend ein Spreizelement, wie beispielsweise ein Keil, eingebracht werden, um eine Verankerung zu sichern. Vorteilhafterweise weisen die gegebenenfalls zwischen den Führungsabschnitten angeordneten Führungselemente Sollbruchstellen auf, welche beim Spreizen der Bohrspitze brechen.

Eine weitere bevorzugte Ausführungsform der Erfindung sieht vor, dass das Bohrwerkzeug einen Bohrkopf mit einer Bohrspitze aufweist, welche gegenüber einer Längsachse gekrümmt ist. Dadurch lassen sich nur an gewünschten Stellen Bohrungen vornehmen. Beispielsweise kann bei einem menschlichen Knochen seitlich am Knochen ein Bohrloch gesetzt werden und durch die gekrümmten Bohrsegmente ein Weiterbohren in die Spangiosa erfolgen, ohne dass die gegenüber liegende Knochenschicht verletzt wird.

Zur weiteren Verringerung der Axialkraft beim Bohren mit dem Bohrsystem weist das Bohrwerkzeug in axialer Richtung gesehen einen Sägezahnabschnitt auf, der sich von dem distalen Ende des Bohrwerkzeugs in proximaler Richtung erstreckt und in einen Schaufelabschnitt übergeht. Folglich wird bei einer Vorschubbewegung der Bohrspitzen der Bohrsegmente in das Material ein Abtragen bewirkt und bei einer Rückbewegung der Bohrsegmente aus dem Material heraus ein Herausfördern des abgetragenen Materials ermöglicht.

Des Weiteren weist der Schneidenabschnitt bevorzugt im mittleren Bereich bezogen auf seine Länge einen Durchmesser auf, der gleich oder größer als der sich in proximaler Richtung daran anschließende Schaufelabschnitt ist. Dadurch verbleibt für den Schaufelabschnitt hinreichend Raum, um das abgetragene Material aus der Bohrung herauszufördern.

Bevorzugt schließt sich an den Schaufelabschnitt des Bohrwerkzeugs in proximaler Richtung ein Schaftabschnitt an, der vorteilhafterweise im Durchmesser gleich oder geringer als der Schaufelabschnitt ist.

Eine weitere bevorzugte Ausführungsform des Bohrwerkzeugs für das Bohrsystem sieht vor, dass eines der Bohrsegmente anstelle des Schneidenabschnitts nur einen Schaufelabschnitt aufweist und vorzugsweise die weiteren Bohrsegmente nur einen Schneidenabschnitt umfassen. Dadurch werden zwei der Bohrsegmente für das Abtragen des Materials und ein Bohrsegment nur für das Herausfördern des Materials eingesetzt, wodurch ein erleichtertes Eintreiben der Bohrsegmente in das Material ermöglicht ist.

Bei der Ausgestaltung des Bohrwerkzeuges ist bevorzugt vorgesehen, dass die Schnittwinkel der Schneiden im Schneidenabschnitt der jeweiligen Bohrsegmente ausgehend vom distalen zum proximalen Ende ansteigend ausgebildet sind sowie vorzugsweise von einem Bereich von circa 70° bis in einen Bereich von circa 130° ansteigen.

Die der Erfindung zugrunde liegende Aufgabe wird des Weiteren durch das Verfahren des unabhängigen Anspruchs 17 zum Antrieb eines Bohrwerkzeuges eines Bohrsystems gelöst, bei dem drei oder mehrere Bohrsegmente des Bohrwerkzeugs in einer vorbestimmten aufeinander folgenden Reihenfolge nach dem Pendelhubprinzip angetrieben werden. Dadurch kann die Herstellung einer Bohrung mit einer nur geringen Axialkraft bewirkt werden. Zudem kann bei einem solchen Antrieb auch ermöglicht sein, dass das Bohrwerkzeug als selbständiger Antrieb in einem Werkstoff wirkt, so dass ein solches Bohrwerkzeug beispielsweise auch bei Erdbohrungen oder dergleichen eingesetzt werden kann.

Die Erfindung sowie weitere vorteilhafte Ausführungsformen und Weiterbildungen derselben werden im Folgenden anhand der in den Zeichnungen dargestellten Beispiele näher beschrieben und erläutert. Die der Beschreibung und den Zeichnungen zu entnehmenden Merkmale können einzeln für sich oder zu mehreren in beliebiger Kombination erfindungsgemäß angewandt werden. Es zeigen:
Figur 1 eine perspektivische Ansicht eines Antriebs eines erfindungsgemäßen Bohrsystems mit Bohrwerkzeug,
Figur 2 eine schematische Seitenansicht von Figur 1,
Figur 3 eine schematische Ansicht von vorne auf den Antrieb gemäß Figur 1 ohne Bohrwerkzeug,
Figuren 4a und 4b schematische Ansichten des Bohrwerkzeuges,
Figuren 5a, 5b und 5c schematische Schnittansichten alternativer Ausführungsformen der Bohrwerkzeuge entlang der Linie V-V in Figur 4a,
Figur 6a eine schematische Seitenansicht eines Bohrkopfes mit vergrößerten Schneidengeometrien,
Figur 6b eine schematische Seitenansicht einer alternativen Ausführungsform eines Bohrkopfes,
Figuren 7a bis 7f schematische Schnittansichten gemäß der Linie V-V in Figur 4a von verschiedenen Geometrien des Bohrwerkzeugs,
Figuren 7g und 7h schematische Schnittansichten entlang der Linie V-V in Figur 4a von einem nicht erfindungsgemäßen Bohrwerkzeug mit zwei Bohrsegmenten,
Figuren 8a und 8b eine schematische Seitenansicht und Vorderansicht einer alternativen Ausführungsform des Werkzeugs gemäß den Figuren 4a und 4b,
Figur 9 eine weitere schematische Seitenansicht einer weiteren alternativen Ausführungsform eines Bohrwerkzeugs gemäß den Figuren 4a und 4b,
Figur 10 eine perspektivische Ansicht einer weiteren alternativen Ausführungsform zu den Figuren 4a und 4b,
Figur 11 eine perspektivische Ansicht einer weiteren alternativen Ausführungsform zu den Figuren 4a und 4b,
Figuren 12a und beine schematische Abfolge von Hubbewegungen der Bohrsegmente des Bohrwerkzeugs gemäß den Figuren 4a und 4b,
Figur 13 eine schematische Seitenansicht einer alternativen Ausführungsform zu Figur 1,
Figur 14 eine schematische Seitenansicht einer weiteren alternativen Ausführungsform des Antriebs zu Figur 13, und
Figur 15 eine schematische Seitenansicht einer weiteren alternativen Ausführungsform des Antriebs zu Figur 13.

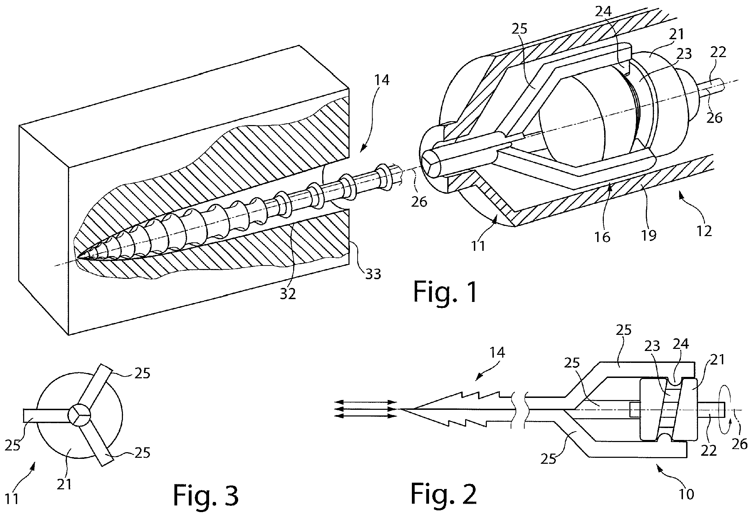

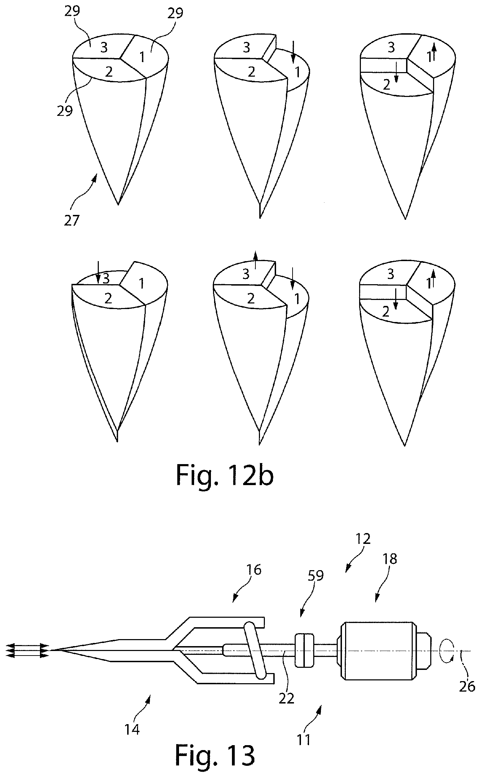

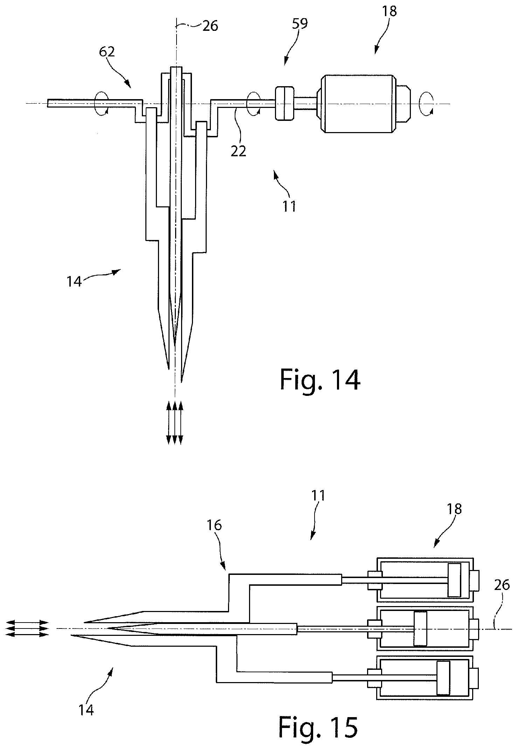

In Figur 1 ist perspektivisch ein Bohrsystem 11 dargestellt, welches ein Bohrinstrument 12 mit einem Bohrwerkzeug 14 umfasst. Das Bohrinstrument 12 ist in dieser Figur 1 lediglich bezüglich eines mechanischen Getriebes 16 dargestellt. Das Bohrinstrument 11 umfasst des Weiteren einen Antrieb 18, der beispielsweise in den Figuren 13 und 14 dargestellt ist. Das Getriebe 16 ist in einem teilweise dargestellten Gehäuse 19 vorgesehen, welches einen nicht näher dargestellten Handgriff oder eine Angriffsfläche zum Betätigen des Bohrinstruments 12 mit der Hand umfasst. Ebenso können Spannflächen oder Angriffsflächen vorgesehen sein, um das Bohrinstrument an einer Vorschubeinrichtung, einem Bohrständer 11 oder einer Maschine oder dergleichen zu befestigen.

Das Getriebe 16 ist mit dem Bohrwerkzeug 14 verbunden und treibt dieses an.

Im Ausführungsbeispiel ist das Getriebe 16 durch ein Kurvengetriebe ausgebildet, bei welchem ein rotierender Zylinder 21 oder eine Kurvenscheibe um eine Antriebsachse 22 angetrieben ist. In einer umlaufenden Nut 23 greift ein Nocken 24 eines Antriebsgestänges 25, das Teil des Getriebes 16 sein kann, an. Die Antriebsgestänge 25 werden in Abhängigkeit des Verlaufs der Nut 23 des Zylinders 21 durch eine Hubbewegung entlang der Längsachse 26 hin und her bewegt. Diese Antriebsgestänge 25 stehen mit dem Bohrwerkzeug 14 in Verbindung.

Das Bohrwerkzeug 14 weist gemäß diesem Ausführungsbeispiel drei Bohrsegmente 29 auf, welche jeweils eine Bohrspitze umfassen und einen Bohrkopf 31 mit einer gemeinsamen Bohrspitze 27 bilden. Durch die Antriebsgestänge 25 wird jedes Bohrsegment 29 mit einer Pendelhubbewegung angetrieben, wie dies schematisch aus Figur 2 ersichtlich ist. Dadurch erfolgt eine Relativbewegung der einzelnen Bohrsegmente 29 zueinander, wodurch ein Vorschub des Bohrwerkzeugs 14 in ein Material oder einen Werkstoff ermöglicht ist. Dadurch kann ohne eine Rotationsbewegung und die Aufbringung eines Drehmomentes eine Bohrung 32 in einen Körper 33 eingebracht werden. Das Bohrwerkzeug 14 kann auch als Antrieb zur Vorwärtsbewegung dienen, so dass ein solches Bohrinstrument auch beispielsweise für Bohrungen im Erdbereich, bei Tunnelgrabungen oder dergleichen einsetzbar ist.

Das Bohrwerkzeug 14 kann alternativ auch mehr als drei Bohrsegmente 29 aufweisen, die einen Bohrkopf 31 bilden.

In Figur 3 ist eine schematische Ansicht von vorne auf das Getriebe 16 gemäß Figur 2 dargestellt. Diese Anordnung des Antriebsgestänges 25 ist an die Geometrie des Bohrwerkzeugs 14 angepasst. Sofern ein dreiteiliges Bohrwerkzeug 14 vorgesehen ist, ist auch ein dreiteiliges Antriebsgestänge 25 vorgesehen. Sofern mehrere Bohrsegmente 29 vorgesehen sind, ist für jedes Bohrsegment 28 ein Antriebsgestänge 25 vorgesehen und im Gehäuse 19 geführt.

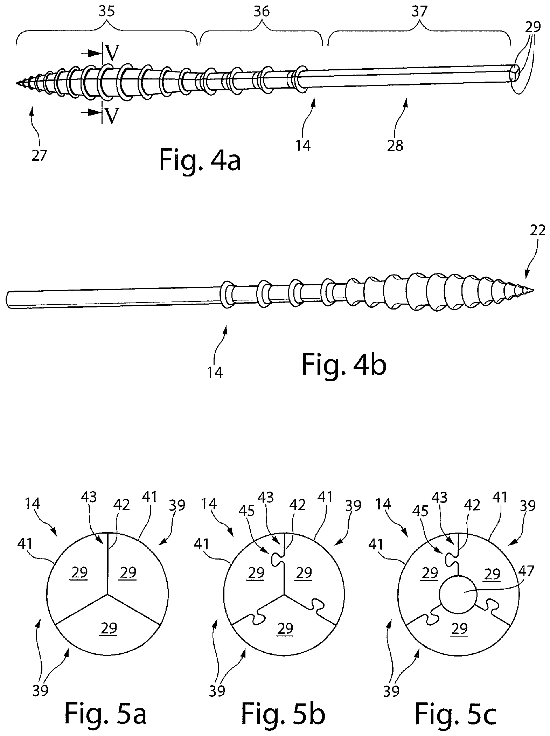

In den Figuren 4a und 4b sind perspektivische Ansichten des Bohrwerkzeuges 14 dargestellt. Dieses Bohrwerkzeug 14 umfasst im Bereich des Bohrkopfes 31 einen Schneidenabschnitt 35, an den sich in proximaler Richtung ein Schaufelabschnitt 36 anschließt. An diesem Schaufelabschnitt 36 beziehungsweise dem Bohrkopf 31 schließt sich darauf folgend ein Schaftabschnitt 37 an. Der Schneidenabschnitt 35 dient zum Materialabtrag und der Einbringung der Bohrung 32. Der Schaufelabschnitt 36 dient zum Herausfördern des abgetragenen Materials aus der Bohrung 32, und der Schaftabschnitt 37 wird in der Länge an die einzubringende Bohrung 32 angepasst.

In Figur 5a ist eine schematische Schnittansicht entlang der Linie V-V in Figur 4a dargestellt. Dieses Bohrwerkzeug 14 ist dreiteilig ausgebildet. Vorteilhafterweise sind drei gleich große Segmentquerschnitte 39 ausgebildet. Jeder Segmentquerschnitt 39 umfasst einen Schneidenbereich 41 sowie jeweils einen daran angrenzenden Führungsabschnitt 42. Die Bohrsegmente 29 stützen sich mit deren Führungsabschnitten 42 gegeneinander ab. Diese liegen in jeweils einer Trennebene 43. Bei einer Ausgestaltung mit gleich großen Segmentquerschnitten 39 liegen die Trennabschnitte 42 in einem Winkel von 120° zueinander beabstandet.

In Figur 5b ist eine modifizierte Ausführungsform des Bohrwerkzeuges 14 zu Figur 5a dargestellt. Die aneinander grenzenden Führungsabschnitte 42 sind durch Führungselemente 45 miteinander verbunden. Diese Führungselemente 45 können sich entlang der Längsachse 26 des Bohrwerkzeugs 14 beispielsweise ausschließlich im Schneidenabschnitt 35 erstrecken, in welchem die größte Kraft auf das Bohrwerkzeug 14 während dem Einbringen der Bohrung 32 gegeben ist. Die Führungselemente 35 können durch Erhöhungen oder Vertiefungen sowohl ohne Hinterschneidungen als auch mit Hinterschneidungen ausgebildet sein, wie letzteres in Figur 5b dargestellt ist.

In Figur 5c ist eine weitere alternative Ausführungsform zu Figur 5b des Bohrwerkzeugs 14 dargestellt. Im mittleren Bereich ist ein Kanal 47 vorgesehen. Dadurch kann beispielsweise eine Spülflüssigkeit, ein Narkotikum oder ein weiteres Medium an den Bohrlochgrund oder in das Bohrloch zugeführt werden. Des Weiteren kann auch ein Funktionsmittel, wie beispielsweise Öl oder Klebstoff, eingebracht werden, welches in der Bohrung 32 und dem daran angrenzenden Bereich verbleiben soll.

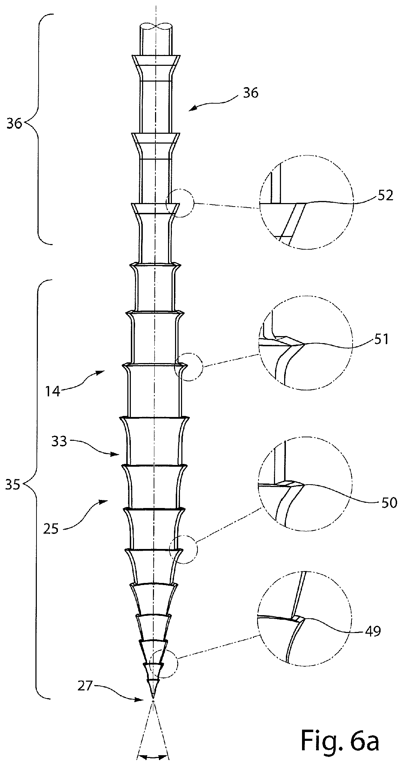

In Figur 6a ist eine schematische Seitenansicht des Schneidenabschnitts 35 und Schaufelabschnitts 36 des Bohrwerkzeugs 14 mit vergrößerten Schneidengeometrien dargestellt. Das Bohrwerkzeug 14 weist beispielsweise eine Bohrspitze 27 mit einem spitzen Winkel in einem Bereich von 10° bis 40°, insbesondere 20° bis 30°, auf. Im mittleren Bereich bezogen auf die Länge des Schneidenabschnitts 35 ist ein Durchmesser vorgesehen, der den Nenndurchmesser der Bohrung 32 bestimmt. Darauf folgend in proximaler Richtung verjüngt sich der Durchmesser im Schneidenabschnitt 35 als auch im Schaftabschnitt 36, um ein erleichtertes Herausfördern des abgetragenen Materials aus der Bohrung 32 zu ermöglichen. Das Bohrwerkzeug 14 weist nahe der Spitze beispielsweise einen Sägezahn 49 mit einem Schnittwinkel von 80° bis 90 ° zur Längsachse auf. Diesem nachgeschaltet kann im mittleren Bereich des Schneidenabschnitts 35 ein Sägezahn 50 mit einem Schnittwinkel von beispielsweise 100° vorgesehen sein. Nahe dem Schaufelabschnitt 36 kann ein Sägezahn 51 einen Schnittwinkel von beispielsweise 120° aufweisen. Im Schaufelabschnitt 36 weist beispielsweise eine Schaufel 52 einen Schnittwinkel von 90° auf. Bei allen Sägezähnen kann ein Keilwinkel von beispielsweise 60° vorgesehen sein.

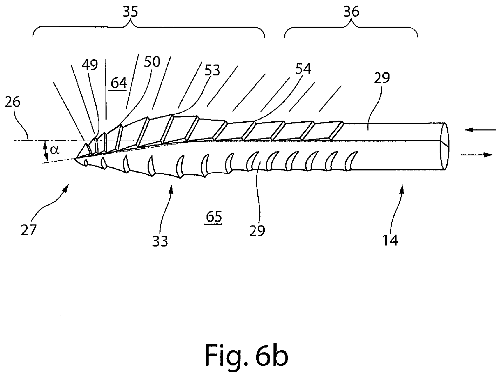

In Figur 6b ist eine alternative Ausführungsform des Bohrwerkzeugs 14 zu Figur 6a dargestellt. Die Längsachse 26 des Bohrwerkzeugs 14 ist ausgehend von einem Schaftabschnitt oder einem Schaufelabschnitt 36 zur Spitze 27 hin in eine Richtung gekrümmt, wobei sich die Krümmung vorzugsweise im Bereich des Schneidenabschnitts 35 erstreckt. Die Krümmung zur Längsachse 26 wird beispielsweise durch den Winkel α ersichtlich. Die jeweiligen Spitzen der Bohrsegmente 29 werden um den Winkel α gegenüber der Längsachse 26 versetzt. Die Kontur des Schneidenabschnitts 36 wird an die Krümmung angepasst, so dass das zumindest eine Bohrsegment 29, welches zur Krümmungsinnenseite 65 gerichtet ist, einen geraden Verlauf oder einen Verlauf mit einer leichten Querschnittsvergrößerung im Schneidenabschnitt 35 aufweist, wobei das zumindest eine Bohrsegment 29, welches zur Krümmungsaußenseite 64 gerichtet ist, im Schneidenabschnitt eine starke Querschnittsvergrößerung aufweist, die vorzugsweise größer ist als die auf der Krümmungsinnenseite 65.

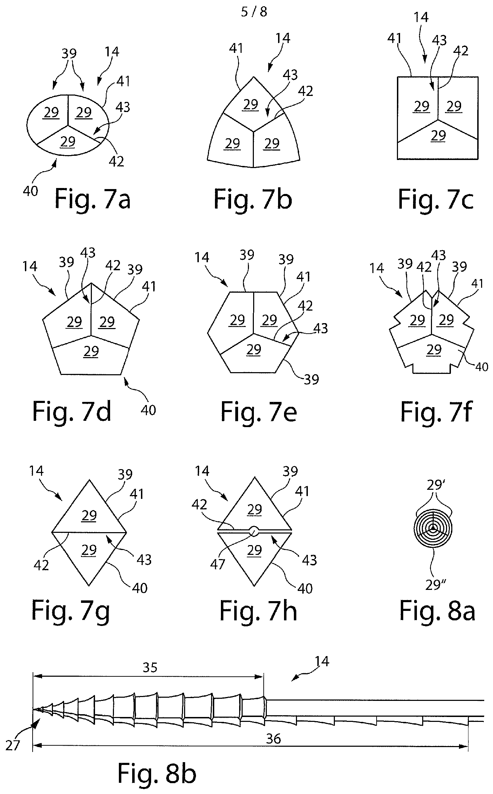

Bei dieser Ausführungsform kann gemäß Figur 6b das unterhalb der Längsachse dargestellte oder vorgesehene Bohrsegment 29 einteilig oder mehrteilig und das oberhalb der Längsachse dargestellte Bohrsegment 24 mehrteilig oder einteilig ausgebildet sein. Beispielsweise kann das untere Bohrsegment 29 zweiteilig und das obere Bohrsegment 29 einteilig ausgebildet sein. Ebenso kann diese Anordnung auch vertauscht sein. Auch können diese Ausgestaltungen sowohl für die zuvor beschriebenen Anordnungsmöglichkeiten gemäß den Figuren 5a bis 5c oder die nachfolgend noch beschriebenen Geometrien gemäß den Figuren 7a bis 8a gelten.

Beispielsweise kann das zumindest eine untere Bohrsegment 29, welches auf der Krümmungsinnenseite 65 liegt, im Schneidenabschnitt 35 die Sägezähne 49 bis 52 gemäß der Ausführungsform in Figur 6a aufweisen. An dem zumindest einen gegenüber liegenden Bohrsegment 29, welches sich entlang der Krümmungsaußenseite 64 erstreckt, kann ebenfalls eine Schneidengeometrie vorgesehen sein, wie diese in Figur 6a beschrieben ist oder aber auch eine alternative Schneidengeometrie vorgesehen sein, wie diese in Figur 6b durch die stilisierten Verlängerungen der Schnittkanten 49, 50, 53 und 54 beispielsweise zur Andeutung des Schnittwinkels dargestellt ist. Beispielsweise ist nahe der Spitze 27 eine Schnittkante 49 vorgesehen, bei welcher der Schnittwinkel kleiner 90°, insbesondere kleiner 80°, zur Längsachse ist. Darauf folgend kann ein Sägezahn 50 mit einem Schnittwinkel von beispielsweise 90° folgen und darauf folgend beispielsweise ein Sägezahn 53 mit einem Schnittwinkel von 100°. Weiterhin in proximaler Richtung kann ein nachfolgender Sägezahn 53 beispielsweise einen Schnittwinkel von 120° und darauf folgend beispielsweise ein Sägezahn 54 im Schaufelbereich 36 einen Schnittwinkel von beispielsweise 140° aufweisen. Die Änderungen im Winkel zur Längsachse 27 der Sägezähne ausgehend von der Spitze 27 zum proximalen Ende hin können in Abhängigkeit der Anzahl der Sägezähne innerhalb des Schneidenabschnitts 35 und/oder des Schaufelabschnitts 36 vorgesehen sein. Des Weiteren kann die Winkelanordnung auch in Abhängigkeit der Krümmung an einer Außenseite des zumindest einen Bohrsegmentes 29 stehen. Die Schnittwinkel der an der Krümmungsaußenseite 64 vorgesehenen Sägezähne sind bevorzugt derart ausgelegt, dass sowohl bei einer Vorschubbewegung als auch einer Rückhubbewegung ein Schnittvorgang ermöglicht ist. Das zumindest eine zur Krümmungsinnenseite 65 ausgerichtete Bohrsegment 29 kann ebenso eine solche Schneidengeometrie wie das zur Krümmungsaußenseite 64 zumindest eine Bohrsegment 29 aufweisen. Alternativ kann dies dahingehend ausgerichtet sein, dass ausschließlich bei einem Rückhub eine Schneidbewegung mit einem gleichzeitigen Abtransport der Späne erfolgt.

Diese sich ändernden Schnittwinkel der einzelnen Sägezähne im Schneidenabschnitt 35 und/oder Schaufelabschnitt 36 entlang der Krümmungsaußenseite 64 des zumindest einen Bohrsegmentes 29 kann auch bei Bohrsegmenten 29 vorgesehen sein, welche sich entlang der Längsachse 26 erstrecken, wie dies beispielsweise in Figur 6a dargestellt ist.

Das drei- oder mehrteilige Bohrwerkzeug 14 kann aufgrund des Pendelhubantriebes von einer runden Geometrie abweichende Geometrien aufweisen. Diese sind beispielsweise in den Figuren 7a bis 7e dargestellt. Bei der Ausführungsform gemäß Figur 7a ist eine ovale Außenkontur des Bohrwerkzeugs 14 gegeben, wobei beispielsweise zwei Segmentquerschnitte 39 in der Größe gleich - jedoch größer als der Segmentquerschnitt 40 - ausgebildet sind. Die Trennebene 43 der einzelnen Bohrspitzen kann jedoch in einem Winkel von 120° zueinander angeordnet sein.

In Figur 7b ist eine Art dreieckförmiger Querschnitt des Bohrkopfes 31 dargestellt. Beispielsweise sind die Segmentquerschnitte 39 gleich groß ausgebildet.

In Figur 7c ist ein quadratischer Querschnitt des Bohrkopfes 31 dargestellt. Die Trennebenen 43 sind nur beispielhaft angeordnet, wobei bevorzugt die Trennebenen 43 nicht in Eckbereiche führen.

In Figur 7d ist eine weitere alternative Ausführungsform einer Querschnittsgeometrie des Bohrkopfes 31 dargestellt. Diese Fünfeckkontur kann beispielsweise in die dargestellten Segmentquerschnitte 39, 40 untergliedert sein.

Eine hexagonale Querschnittsgeometrie für einen Bohrkopf 31 ist beispielsweise in Figur 7e dargestellt. Bei dieser Ausführungsform sind die Trennebenen 43 derart vorgesehen, dass gleich große Segmentquerschnitte 39 entstehen.

Eine weitere alternative Ausführungsform für einen Bohrkopf 31 ist in Figur 7f dargestellt. Beispielsweise sind die Eckbereiche ausgeschnitten. Dies kann rund, eckig oder durch weitere Freiformen gegeben sein. Diese Anordnung kann auch bei den zuvor beschriebenen Figuren vorgesehen sein.

Der äußere Schneidenverlauf für jedes Bohrsegment kann auch voneinander abweichende Verläufe aufweisen, die jedoch in dem Übergangsbereich zur Trennebene aneinander angrenzen.

Bei allen Ausführungsformen können jedoch Trennebenen 43 auch abweichend davon vorgesehen sein, so dass entweder zwei gleich große Segmentquerschnitte oder drei unterschiedlich große Segmentquerschnitte ausgebildet sein können.

Es versteht sich, dass anstelle eines dreiteiligen Bohrwerkzeugs 14 auch ein darüber hinaus mehrteiliges Bohrwerkzeug 14 mit mehreren Bohrsegmenten 29 ausgebildet sein kann.

In Figur 7g ist eine schematische Schnittdarstellung eines zweiteiligen, nicht erfindungsgemäßen Bohrwerkzeuges 14 dargestellt, welches eine von einer runden Geometrie abweichende Geometrie des Bohrkopfes 31 aufweist. Das aus zwei Bohrsegmenten 29 bestehende Bohrwerkzeug 14 umfasst jeweils einander zugeordnete Führungsabschnitte 42, die in einer gemeinsamen Trennebene 43 liegen. Ein Schneidenbereich 41 geht in den jeweiligen Führungsabschnitt 42 über. Dadurch kann bspw. eine trapezförmige oder drachenförmige Geometrie geschaffen werden, die von einer runden Geometrie abweicht, wie dies in Figur 7g dargestellt ist. Beim Ausführungsbeispiel gemäß Figur 7g sind die Segmentquerschnitte 39 und 40 gleich groß. Alternativ kann vorgesehen sein, dass diese in der Größe auch voneinander abweichen, wobei bevorzugt vorgesehen ist, dass die Führungsabschnitte 42 gleichlang ausgebildet sind. Alternativ kann auch bei der Wahl einer bestimmten Geometrie vorgesehen sein, dass der Führungsabschnitt 42 von dem einen Bohrsegment 29 ein oder beidseitig gegenüber dem anderen Bohrsegment 29 hervorsteht.

In Figur 7h ist eine alternative, nicht erfindungsgemäße Ausführungsform zu Figur 7g dargestellt. Diese Ausführungsform weist bspw. zentral einen Kanal 47 auf, durch welchen fließförmige Medien hindurchgeführt werden können, wie dies bspw. auch beim Ausführungsbeispiel gemäß Figur 5c beschrieben ist.

Bei einem zweiteiligen Bohrwerkzeug 14 mit zwei Bohrsegmenten 29 weist der Bohrkopf 31 einen von einer runden Geometrie abweichenden Außenumfang auf, der in der konkreten Ausgestaltung jedoch vielfältig ausgebildet sein kann. Bspw. können auch die Geometrien gemäß Figur 7a bis 7f durch ein Bohrwerkzeug 14 mit zwei Bohrsegmenten 29 ausgebildet sein.

In Figur 8a ist eine schematische Ansicht von vorne auf eine alternative Ausführungsform eines Bohrwerkzeuges 14 gemäß Figur 8b dargestellt. Bei diesem dreiteiligen Bohrwerkzeug 14 ist vorgesehen, dass die Bohrsegmente 29' jeweils einen Schneidenabschnitt 35 und nur einen Schaftabschnitt 37 aufweisen, das heißt, dass der Schaufelabschnitt 36 nicht vorgesehen ist. Das dritte Bohrsegment 29" weist dafür nur einen Schaufelabschnitt 36 auf, der sich in proximaler Richtung weit über den Schneidenabschnitt 35 der Bohrsegmente 29' erstreckt. Dadurch haben zwei Bohrsegmente eine schneidende Funktion und ein Bohrsegment eine Förderfunktion.

Bei dieser Ausführungsform kann des Weiteren vorgesehen sein, dass die beiden Bohrsegmente 29' zusammen einen Halbkreis bilden und das dritte Bohrsegment 29" einen zweiten Halbkreis bildet, das heißt, dass der Segmentquerschnitt 39 der Bohrspitze 29' doppelt so groß ist wie der Segmentquerschnitt 40 der Bohrspitze 29".

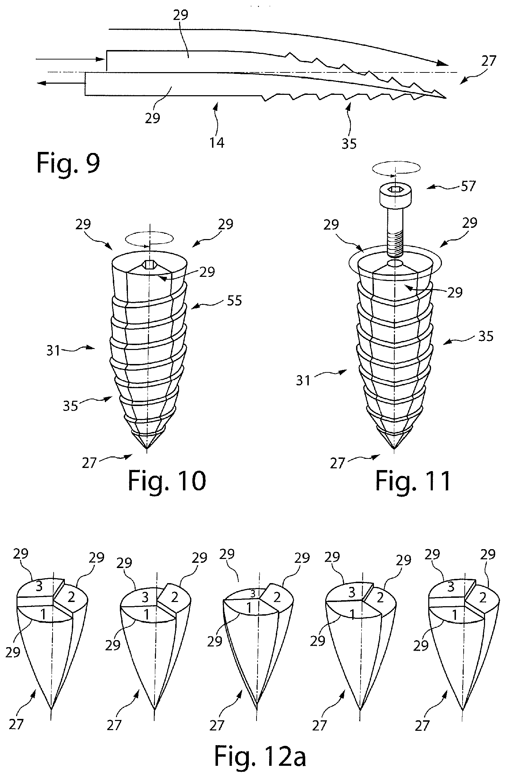

In Figur 9 ist eine weitere alternative Ausführungsform eines Bohrwerkzeuges 14 vorgesehen. Die Bohrsegmente 29 sind gegenüber einer Längsachse 26 gekrümmt ausgebildet, so dass eine gekrümmte Bohrung oder ein schräges oder gekrümmtes Loch gebohrt werden kann. Insbesondere kann eine solche Ausführungsform auch für das Herstellen eines Hohlraumes in einem Knochen, insbesondere Oberschenkelknochen, zum anschließenden Einsetzen einer Hüftgelenkprothese oder dergleichen genutzt werden. Vorteilhafterweise ist das Bohrwerkzeug 14 dabei nicht nur als Bohr-, sondern auch als Raspelwerkzeug ausgebildet.

In Figur 10 ist eine weitere alternative Ausführungsform des Bohrwerkzeugs 14 dargestellt. Die Bohrspitze 29 ist dabei von einer Bohrlanze oder einem Schaftabschnitt 37 lösbar und weist bevorzugt Schneiden auf, die einem Gewindeverlauf 55 entsprechen. Dadurch kann nach dem Bohren durch eine Drehbewegung des Bohrsegments 29 eine Verankerung erzielt werden. Der in dem Material verbleibende Bohrkopf 31 kann als Dübel funktionieren, um ein weiteres Verankerungselement aufzunehmen.

Eine weitere alternative Ausführungsform eines lösbaren Bohrkopfes 31 ist in Figur 11 dargestellt. Hierbei kann der Bohrkopf 31 als Spreizelement verwendet werden, indem beispielsweise ein Spannelement 57 in Form einer Schraube oder eines Keiles eingebracht wird, um die drei Bohrsegmente 29 des Bohrkopfes 31 aufzuweiten.

In Figur 12 ist eine schematische Abfolge einer Pendelhubbewegung von drei Bohrsegmenten 29 des Bohrwerkzeuges 14 dargestellt, um einen Vorschub oder ein Eindringen in einen Körper 33 zur Herstellung der Bohrung 32 zu bewirken. Zunächst wird beispielsweise das Bohrsegment 1 mit einem Vorschub beaufschlagt. Anschließend wird gemäß Bild 2 der Figur 12 das Bohrsegment 3 eingetrieben und darauffolgend die Bohrsegmente 1 durch eine Hubbewegung geringfügig zurück genommen, bevor gemäß Bild 4 das Bohrsegment 3 ebenfalls zurück genommen wird, um anschließend das Bohrsegment 2 mit einer Vorschubbewegung zu beaufschlagen. Darauf folgend wird das Bohrsegment 1 wieder in die Bohrung 32 hinein bewegt, und die gleiche Abfolge der einzelnen Pendelhübe, die auf die drei Bohrsegmente 29 wirken, beginnt von vorne.

Alternative Reihenfolgen sind ebenso denkbar.

In Figur 12b ist beispielsweise eine von mehreren alternativen Reihenfolgen dargestellt. Dabei ist vorgesehen, dass beispielsweise aufeinander folgend das erste Bohrsegment axial nach vorne und anschließend wieder zurück bewegt wird, wobei während der Rückbewegung des ersten Bohrsegments 29 das benachbarte Bohrsegment 29 eine Vorwärtsbewegung erfährt. Diese aufeinander folgende hin und her Bewegung wird beispielsweise im Uhrzeigersinn durchlaufen, wodurch bei Schritt 5 wieder ein neuer Bearbeitungszyklus eingeleitet wird. Schritt 6 entspricht wiederum dem Schritt 3. Ein Schritt 7 würde dem Schritt 4 entsprechen und ein Schritt 8 dem Schritt 5 usw. Alternativ kann die Ansteuerung der Bohrsegmente 29 auch entgegen dem Uhrzeigersinn erfolgen.

In Figur 13 ist eine alternative Ausführungsform eines Getriebes 16 mit einem Antrieb 18 gemäß Figur 1 dargestellt. Anstelle eines Zylinderkolbens 21 mit einer umlaufenden Nut 25 weist das Getriebe 16 eine Taumelscheibe auf, um das Antriebsgestänge 25 anzutreiben. In dieser Ausführungsform ist der Motor 18 dargestellt, der über eine Kupplung 59 an dem Getriebe 16 angreift. Dadurch kann beispielsweise ein einfacher Austausch des Bohrwerkzeugs 14 oder des Motors 18 gegeben sein, wobei in diesem Ausführungsbeispiel der jeweilige Schaft des Bohrwerkzeuges 14 und des Antriebsgestänges 25 einteilig ausgebildet sind. Alternativ kann dazwischen liegend eine Schnittstelle geschaffen sein, so dass nur das Bohrwerkzeug 14 austauschbar zum Getriebe 16 vorgesehen ist.

Bei der Ausführungsform gemäß Figur 13 liegt die Antriebsachse 22 des Motors 18 in der Längsachse 26 des Bohrwerkzeugs 14 und Rotationsachse der Taumelscheibe.

In Figur 14 ist eine alternative Ausführungsform zu Figur 13 dargestellt. Bei dieser Ausführungsform ist der Motor 18 mit seiner Antriebsachse 22 senkrecht zur Längsachse 26 des Bohrwerkzeugs 14 vorgesehen und kann beispielsweise über einen Kurbelantrieb 62, insbesondere eine Kurbelwelle 62, das Bohrwerkzeug 14 entsprechend antreiben.

In Figur 15 ist eine weitere alternative Ausführungsform des Bohrsystems 11 zu Figur 13 dargestellt. Diese alternative Ausführungsform des Bohrsystems 11 umfasst einen pneumatischen Antrieb 18, der beispielsweise drei einzelne Pneumatikzylinder aufweist, welche jeweils ein Bohrsegment 29 antreiben. Die einzelnen Pneumatikzylinder werden über Schaltventile angesteuert, wobei die Schaltsignale aufeinander synchronisiert sind, um die entsprechenden Vorschubbewegungen der Bohrsysteme 29 anzusteuern. Alternativ kann das Bohrwerkzeug 14 mit beispielsweise drei Bohrsegmenten 29 drei einzelne Hydraulikzylinder umfassen, die aufeinander synchronisiert angesteuert werden. Alternativ kann vorgesehen sein, dass der hydraulische Antrieb anstelle eines Elektromotors vorgesehen ist und eine rotierende Welle antreibt, mit einem Antrieb 16 zusammenwirkt, wie dies beispielsweise in Figur 13 dargestellt ist.

## Patentansprüche

1. Bohrsystem mit einem Bohrinstrument (12),
- welches einen Antrieb (18) aufweist, der ein Bohrwerkzeug (14) antreibt, das eine geteilte Bohrspitze (27) umfasst, **dadurch gekennzeichnet,**
- **dass** das Bohrwerkzeug (14) drei oder mehr Bohrsegmente (29) aufweist, die einen Bohrkopf (31) mit einer gemeinsamen Bohrspitze (27) bilden, wobei die einzelnen Bohrsegmente (29) in einer vorbestimmten aufeinanderfolgenden Reihenfolge nach dem Pendelhubprinzip angetrieben werden.

2. Bohrsystem nach Anspruch 1, **dadurch gekennzeichnet, dass** eine Antriebsachse (22) des Antriebs (16, 18) in der Längsachse (26) des Bohrwerkzeugs (14) liegt und vorzugsweise ein Getriebe (16), insbesondere eine Taumelscheibe oder ein Kurvenantrieb, ansteuerbar ist oder dass eine Antriebsachse (22) des Antriebs (18) quer zur Längsachse (26) des Bohrwerkzeugs (14) liegt und vorzugsweise ein Getriebe (16), insbesondere ein Kurbelantrieb (62), ansteuerbar ist.

3. Bohrsystem nach Anspruch 1, **dadurch gekennzeichnet, dass** jedes Bohrsegment (29) des Bohrwerkzeugs (14) mit einem elektrischen, pneumatischen oder hydraulischen Antrieb (18) separat ansteuerbar ist.

4. Bohrsystem nach Anspruch 1, **dadurch gekennzeichnet, dass** jedes Bohrsegment (29) einen Schneidenabschnitt (41) und zumindest zwei Führungsabschnitte (42) aufweist, wobei zwei an den Schneidenabschnitten (41) angrenzende Führungsabschnitte (42) mit korrespondierenden Führungsabschnitten (42) der benachbarten Bohrsegmente (29) aneinander anliegen.

5. Bohrsystem nach Anspruch 4, **dadurch gekennzeichnet, dass** die aneinander liegenden Führungsabschnitte (42) der Bohrsegmente (29) in einer radial ausgerichteten Trennebene (43) aneinander anliegen und vorzugsweise die Trennebene (43) der aneinander liegenden Führungsabschnitte (42) in gleichen Winkeln zueinander angeordnet sind oder dass an den aneinander liegenden Führungsabschnitten (42) der Bohrsegmente (29) Führungselemente (45) vorgesehen sind, durch welche die Führungsabschnitte (42) miteinander in Verbindung stehen.

6. Bohrsystem nach einem der Ansprüche 4 oder 5, **dadurch gekennzeichnet, dass** der Schneidenabschnitt (41) von dem einen Bohrsegment (29) mit denen der weiteren Bohrsegmente (29) eine runde, ovale, dreieck- oder mehreckförmige Schneidengeometrie aufweist.

7. Bohrsystem nach Anspruch 1, **dadurch gekennzeichnet, dass** jedes Bohrsegment (29) einen Schneidenabschnitt (41) und einen Führungsabschnitt (42) aufweist, die aneinander liegen und vorzugsweise die aneinander liegenden Führungsabschnitte (42) der Bohrsegmente (29) in ein geradlinig ausgerichteten, einer gekrümmten oder abgewinkelten Trennebene (43) einander anliegen oder an den aneinander liegenden Führungsabschnitten (42) der Bohrsegmente (29) Führungselemente (45) vorgesehen sind, durch welche die Führungsabschnitte (42) miteinander in Verbindung stehen.

8. Bohrsystem nach einem der vorhergehenden Ansprüche, **dadurch gekennzeichnet, dass** der Schneidenabschnitt (41) des einen Bohrsegmentes (29) mit dem des weiteren Bohrsegments (29) eine ovale, dreieck- oder mehreckförmige Schneidengeometrie aufweist.

9. Bohrsystem nach Anspruch 1, **dadurch gekennzeichnet, dass** das Bohrwerkzeug (14) zumindest ein Bohrsegment (29) mit einem größeren Segmentquerschnitt (39) als das oder die weiteren Bohrsegmente (29) umfasst, insbesondere bei drei Bohrsegmenten (29) einen Segmentquerschnitt von 50 % des Gesamtquerschnitts der Bohrspitze (27) umfasst und die beiden weiteren Segmentquerschnitte jeweils einen Segmentquerschnitt von 25 % des Gesamtquerschnitts aufweisen.

10. Bohrsystem nach Anspruch 1, **dadurch gekennzeichnet, dass** das Bohrwerkzeug (14) für jedes Bohrsegment (29) eine Bohrlanze umfasst, an der zumindest der Schneidenabschnitt (35) lösbar angeordnet ist.

11. Bohrsystem nach Anspruch 1, **dadurch gekennzeichnet, dass** die Bohrsegmente (29) zumindest im Bereich der Bohrspitze (27) mit einem Gewindeverlauf (55) versehen sind, welcher sich radial über die Bohrsegmente (29) erstreckt.

12. Bohrsystem nach Anspruch 1 oder 2, **dadurch gekennzeichnet, dass** das Bohrwerkzeug (14) zur zentrischen Aufnahme eines Spreizelementes (57) ausgebildet ist und vorzugsweise die zwischen dem Führungsabschnitt (42) ausgebildeten Führungselemente (45) eine Sollbruchstelle aufweisen, welche beim Spreizen der Bohrsegmente bricht.

13. Bohrsystem nach Anspruch 1 oder 2, **dadurch gekennzeichnet, dass** das Bohrwerkzeug (14) eine gekrümmte Bohrspitze (27) aufweist, bei der die Bohrsegmente (29) gegenüber der Längsachse (26) des Bohrwerkzeugs (14) abweichend ausgerichtet sind.

14. Bohrsystem nach Anspruch 1 oder 2, **dadurch gekennzeichnet, dass** die Bohrsegmente (29) in axialer Richtung des Bohrwerkzeugs (14) gesehen einen Schneidenabschnitt (35) aufweist, der sich vom distalen Ende der Bohrspitze (27) in proximaler Richtung erstreckt und in einen Schaufelabschnitt (36) übergeht und vorzugsweise der Schneidenabschnitt (35) in einem mittleren Bereich einen Durchmesser aufweist, der gleich oder größer als der Durchmesser des Schaufelabschnitts (36) ist und insbesondere sich an den Schaufelabschnitt (36) in proximaler Richtung ein Schaftabschnitt (37) anschließt oder an wenigstens einem der Bohrsegmente (29) anstelle des Schneidenabschnitts (35) nur ein Schaufelabschnitt (36) und vorzugsweise an den weiteren Bohrsegmenten (29) des Bohrwerkzeuges (14) jeweils nur ein Schneidenabschnitt (35) vorgesehen ist.

15. Bohrsystem nach Anspruch 14, **dadurch gekennzeichnet, dass** ein Schnittwinkel der Sägezähne (49 bis 52) im Schneidenabschnitt (35) des Bohrsegments (29) ausgehend vom distalen zum proximalen Ende des Bohrwerkzeuges (14) ansteigend ausgebildet ist und vorzugsweise in einem Bereich von circa 70° bis 140° ansteigt.

16. Bohrwerkzeug zur Verwendung in einem Bohrsystem nach einem der Ansprüche 1 bis 15, **dadurch gekennzeichnet, dass** das Bohrwerkzeug (14) drei oder mehr Bohrsegmente (29) aufweist, die einen Bohrkopf (31) mit einer gemeinsamen Bohrspitze (27) bilden, wobei die einzelnen Bohrsegmente (29) in einer vorbestimmten aufeinanderfolgenden Reihenfolge nach dem Pendelhubprinzip antreibbar sind.

17. Verfahren zum Antrieb eines Bohrwerkzeuges eines Bohrsystems nach einem der Ansprüche 1 bis 15, **dadurch gekennzeichnet, dass** die einzelnen Bohrsegmente (29) in einer vorbestimmten aufeinander folgenden Reihenfolge nach dem Pendelhubprinzip angetrieben werden.

## Claims

1. A drilling system having a drilling instrument (12)
- which has a drive mechanism (18) that drives a drilling tool (14) comprising a segmental drill bit (27), **characterised in that**
- the drilling tool (14) has three or more drilling segments (29) forming a drill head (31) which shares a common drill bit (27), the individual drilling segments (29) being driven in a predetermined sequence of motions according to the principle of an oscillating stroke operation.

2. The drilling system as claimed in claim 1, **characterised in that** a drive axis (22) of the drive mechanism (16, 18) is situated in the longitudinal axis (26) of the drilling tool (14) and preferably a gear unit (16), in particular a swash plate or a cam drive, may be activated, or **in that** a drive axis (22) of the drive mechanism (18) is situated transversely to the longitudinal axis (26) of the drilling tool (14) and preferably a gear unit (16), in particular a crank mechanism (62), may be activated.

3. The drilling system as claimed in claim 1, **characterised in that** each drilling segment (29) of the drilling tool (14) may be separately activated by means of an electric, pneumatic, or hydraulic drive mechanism (18).

4. The drilling system as claimed in claim 1, **characterised in that** each drilling segment (29) has a cutting edge portion (41) and at least two guide portions (42), with two guide portions (42) adjoining the cutting edge portions (41) abutting respectively against corresponding guide portions (42) of the adjacent drilling segments (29).

5. The drilling system as claimed in claim 4, **characterised in that** said abutting guide portions (42) of the drilling segment (29) abut against each other in a radially oriented separation plane (43), respectively, and **in that** preferably the respective separation planes (43) of the abutting guide portions (42) are disposed at equal angles from one another, or **in that** said abutting guide portions (42) of the drilling segments (29) have guide elements (45) provided thereon by means of which the guide portions (42) are in contact with one another.

6. The drilling system as claimed in any one of claims 4 or 5, **characterised in that** the cutting edge portion (41) of the one drilling segment (29), taken in conjunction with those of the other drilling segments (29), has a round, oval, triangular, or polygonal cutting edge geometry.

7. The drilling system as claimed in claim 1, **characterised in that** each drilling segment (29) has a cutting edge portion (41) and a guide portion (42) which adjoin each other and **in that** preferably the abutting guide portions (42) of the drilling segment (29) abut against each other in a rectilinearly oriented, a curved or an angled separation plane (43), or **in that** the abutting guide portions (42) of the drilling segments (29) have guide elements (45) provided thereon by means of which the guide portions (42) are in contact with one another.

8. The drilling system as claimed in any of the preceding claims, **characterised in that** the cutting edge portion (41) of the one drilling segment (29), taken in conjunction with that of the further drilling segment (29), forms an oval, a triangular, or a polygonal cutting edge geometry.

9. The drilling system as claimed in claim 1, **characterised in that** the drilling tool (14) comprises at least one drilling segment (29) which has a larger segment cross-section (39) than the further drilling segment(s) (29), in particular, in the case of three drilling segments (29), comprises a segment cross-section amounting to 50 % of the overall cross-section of the drill bit (27), the two further segment cross-sections having each a segment cross-section of amounting to 25 % of said overall cross-section.

10. The drilling system as claimed in claim 1, **characterised in that** for each drilling segment (29), the drilling tool (14) comprises a drilling lance on which at least the cutting edge portion (35) is detachably arranged.

11. The drilling system as claimed in claim 1, **characterised in that** at least in the region of the drill bit (27), the drilling segments (29) are provided with a threaded portion (55) extending radially over said drilling segments (29).

12. The drilling system as claimed in claim 1 or 2, **characterised in that** the drilling tool (14) is arranged for receiving a spreading element (57) in its centre and **in that** preferably the guiding elements (45) formed between the guide portions (42) have a breaking point which breaks when the drilling segments are being spread apart.

13. The drilling system as claimed in claim 1 or 2, **characterised in that** the drilling tool (14) has a curved drill bit (27) in which the drilling segments (29) are oriented in a manner deviating from the longitudinal axis (26) of the drilling tool (14).

14. The drilling system as claimed in claim 1 or 2, **characterised in that** the drilling segments (29), when considered in an axial direction of the drilling tool (14), have a cutting edge portion (35) which, from the distal end of the drill bit (27), extends in a proximal direction and transitions into a blade portion (36), and **in that** preferably the cutting edge portion (35) in a central region thereof has a diameter which is equal to, or larger than, the diameter of the blade portion (36), and **in that** in particular said blade portion (36) is followed, in a proximal direction, by a shaft portion (37), or **in that** on at least one of the drilling segments (29) only a blade portion (36) is provided instead of the cutting edge portion (35) and **in that** on each of the further drilling segments (29) of the drilling tool (14) only a cutting edge portion (35) is provided.

15. The drilling system as claimed in claim 14, **characterised in that** a cutting angle of the saw teeth (49 to 52) present in the cutting edge portion (35) of the drilling segment (29) is formed so as to augment from the distal end to the proximal end of the drilling tool (14) and preferably augments in a range of between approximately 70° and 140°.

16. A drilling tool to be utilised in a drilling system as claimed in any one of claims 1 to 15, **characterised in that** the drilling tool (14) has three or more drilling segments (29) forming a drill head (31) which shares a common drill bit (27), the individual drilling segments (29) being capable of being driven in a predetermined sequence of motions according to the principle of an oscillating stroke operation.

17. A method of driving a drilling tool of a drilling system as claimed in any one of claims 1 to 15, **characterised in that** the individual drilling segments (29) are driven in a predetermined sequence of motions according to the principle of an oscillating stroke operation.

## Revendications

1. Système de forage pourvu d'un instrument de forage (12),
- lequel présente un entraînement (18) qui entraîne un outil de forage (14) qui comprend une pointe de forage (27) constituée de plusieurs pièces, **caractérisé en ce que**
- l'outil de forage (14) présente trois segments de forage (29) ou plus, lesquels forment une tête de forage (31) ayant une pointe de forage commune (27), les différents segments de forage (29) étant entraînés selon le principe du mouvement pendulaire dans un ordre de succession prédéterminé.

2. Système de forage selon la revendication 1, **caractérisé en ce qu'**un axe d'entraînement (22) de l'entraînement (16, 18) se situe dans l'axe longitudinal (26) de l'outil de forage (14) et que de préférence un mécanisme (16), en particulier un disque en nutation ou un entraînement à cames, peut être activé, ou **en ce qu'**un axe d'entraînement (22) de l'entraînement (18) se situe transversalement à l'axe longitudinal (26) de l'outil de forage (14) et que de préférence un mécanisme (16), en particulier un entraînement par manivelle (62), peut être activé.

3. Système de forage selon la revendication 1, **caractérisé en ce que** chaque segment de forage (29) de l'outil de forage (14) peut être activé séparément grâce à un entraînement (18) électrique, pneumatique ou hydraulique.

4. Système de forage selon la revendication 1, **caractérisé en ce que** chaque segment de forage (29) présente une partie d'arête de coupe (41) et au moins deux parties de guidage (42), deux parties de guidage (42) contiguës aux parties d'arête de coupe (41) reposant contre des parties de guidage (42) correspondantes des segments de forage (29) voisins.

5. Système de forage selon la revendication 4, **caractérisé en ce que** les parties de guidage (42) des segments de forage (29) qui reposent les unes contre les autres le font dans un plan de séparation (43) qui est orienté de manière radiale et **en ce que** de préférence les plans de séparation (43) des parties de guidage (42) qui reposent les unes contre les autres sont disposés les uns par rapport aux autres selon le même angle ou **en ce que** sur les parties de guidage (42) des segments de forage (29) qui reposent les unes contre les autres sont prévus des éléments de guidage (45) grâce auxquels les parties de guidage (42) sont en contact les unes avec les autres.

6. Système de forage selon l'une quelconque des revendications 4 ou 5, **caractérisé en ce que** la partie d'arête de coupe (41) d'un segment de forage donné (29) présente avec celles des autres segments de forage (29) une géométrie d'arête de coupe de forme ronde, ovale, triangulaire ou polygonale.

7. Système de forage selon la revendication 1, **caractérisé en ce que** chaque segment de forage (29) présente une partie d'arête de coupe (41) et une partie de guidage (42) qui reposent l'une contre l'autre et **en ce que** de préférence les parties de guidage (42) des segments de forage (29) qui reposent les unes contre les autres le font dans un plan de séparation (43) qui est orienté de manière rectiligne, qui est incurvé ou qui est angulaire, ou **en ce que** sur les parties de guidage (42) des segments de forage (29) qui reposent les unes contre les autres sont prévus des éléments de guidage (45) grâce auxquels les parties de guidage (42) sont en contact les unes avec les autres.

8. Système de forage selon l'une quelconque des revendications précédentes, **caractérisé en ce que** la partie d'arête de coupe (41) d'un segment de forage donné (29) présente avec celle de l'autre segment de forage (29) une géométrie d'arête de coupe de forme ovale, triangulaire ou polygonale.

9. Système de forage selon la revendication 1, **caractérisé en ce que** l'outil de forage (14) comprend au moins un segment de forage (29) ayant une section transversale de segment (39) qui est plus grande que le ou les autres segments de forage (29), en particulier comprend, dans le cas de trois segments de forage (29), une section transversale de segment faisant 50 % de la section transversale totale de la pointe de forage (27) et les deux autres sections transversales de segment présentant respectivement une section transversale de segment faisant 25 % de la section transversale totale.

10. Système de forage selon la revendication 1, **caractérisé en ce que** l'outil de forage (14) comprend, pour chaque segment de forage (29), une lance de forage sur laquelle au moins la partie d'arête de coupe (35) est disposée de manière amovible.

11. Système de forage selon la revendication 1, **caractérisé en ce que** les segments de forage (29) sont pourvus, au moins dans la zone de la pointe de forage (27), d'un parcours de filetage (55) qui s'étend de manière radiale le long des segments de forage (29).

12. Système de forage selon la revendication 1 ou 2, **caractérisé en ce que** l'outil de forage (14) est formé pour recevoir de manière centrale un élément d'écartement (57) et **en ce que** de préférence les éléments de guidage (45) formés entre les parties de guidage (42) présentent un point destiné à la rupture qui se rompt lorsque les segments de forage sont écartés.

13. Système de forage selon la revendication 1 ou 2, **caractérisé en ce que** l'outil de forage (14) présente une pointe de forage (27) incurvée dans laquelle les segments de forage (29) sont orientés de manière différente par rapport à l'axe longitudinal (26) de l'outil de forage (14).

14. Système de forage selon la revendication 1 ou 2, **caractérisé en ce que** les segments de forage (29), vus en direction axiale de l'outil de forage (14), présentent une partie d'arête de coupe (35) qui s'étend en direction proximale depuis l'extrémité distale de la pointe de forage (27) et qui se prolonge par une partie pelle (36), et **en ce que** de préférence la partie d'arête de coupe (35) présente, dans une zone centrale, un diamètre qui est aussi grand ou plus grand que le diamètre de la partie pelle (36), et en particulier que la partie pelle (36) est suivie en direction proximale par une partie queue (37), ou **en ce que** sur au moins un des segments de forage (29) est prévue seulement une partie pelle (36) à la place de la partie d'arête de coupe (35), et que de préférence sur les autres segments de forage (29) de l'outil de forage (14) est respectivement prévue seulement une partie d'arête de coupe (35).

15. Système de forage selon la revendication 14, **caractérisé en ce qu'**un angle de coupe des dents de scie (49 à 52) situées dans la partie d'arête de coupe (35) du segment de forage (29) est formé de manière à s'accroître depuis l'extrémité distale vers l'extrémité proximale de l'outil de forage (14) et s'accroît de préférence dans une plage comprise entre environ 70° et 140°.

16. Outil de forage destiné à être utilisé dans un système de forage selon l'une quelconque des revendications 1 à 15, **caractérisé en ce que** l'outil de forage (14) présente trois segments de forage (29) ou plus, lesquels forment une tête de forage (31) ayant une pointe commune (27), les différents segments de forage (29) pouvant être entraînés selon le principe du mouvement pendulaire dans un ordre de succession prédéterminé.

17. Procédé destiné à entraîner un outil de forage d'un système de forage selon l'une quelconque des revendications 1 à 15, **caractérisé en ce que** les différents segments de forage (29) sont entraînés selon le principe du mouvement pendulaire dans un ordre de succession prédéterminé.
